# EUROPEAN PATENT APPLICATION

(11) **EP 4 299 024 A1**
(43) Date of publication of application: **03.01.2024**
(21) Application number: 23181887.3
(22) Date of filing: 27.06.2023
(51) Int. Cl.: A61B 18/14, A61B 17/29, A61B 18/00

(54) **ENDOSCOPIC TREATMENT TOOL AND WATER SUPPLY MEMBER**

(30) Priority: 29.06.2022 JP 2022104771
(71) Applicant: FUJIFILM Corporation, Tokyo 106-8620 (JP)
(72) Inventor: ISHIKAWA, Ryo, Kanagawa, 2588538 (JP)
(74) Representative: Kudlek, Franz Thomas

(57) **Abstract**

An endoscopic treatment tool includes a treatment tool main body of which a distal end part is configured to be opened and closed and which extends in a first direction; an operation member provided on a proximal end side of the treatment tool main body and used for an opening and closing operation of the distal end part; and an accommodating member, accommodating the treatment tool main body and having a through-hole allowing fluid to be supplied inside. The accommodating member and the treatment tool main body move relative to each other in the first direction so as to form a first state in which the treatment tool main body protrudes from a first opening on a distal end side of the accommodating member or a second state in which a part of a protrusion range of the treatment tool main body is retracted toward the proximal end side.

## Description

### BACKGROUND OF THE INVENTION

### 1. Field of the Invention

The present invention relates to an endoscopic treatment tool and a water supply member.

### 2. Description of the Related Art

JP2007-68596A describes a high-frequency treatment tool capable of restricting an amount of protrusion of a high-frequency knife consisting of a rod-shaped electrode, ensuring stability of the high-frequency knife during treatment such as incision, and performing fluid feeding and drainage during the treatment.

JP2005-224426A describes an endoscopic treatment tool that is provided with a treatment portion provided at a distal end of a treatment tool insertion part inserted into a channel of an endoscope and including a pair of forceps cups and that is provided with a liquid supply pipe line and a liquid supply lumen supplying a liquid from a distal end of the treatment portion toward a target site.

JP2007-20969A describes an endoscopic high-frequency treatment tool with a water supply function having a configuration in which a distal end of a water supply tube inserted into and disposed in a flexible sheath is connected to a nozzle pipe disposed at a distal end of the flexible sheath.

### SUMMARY OF THE INVENTION

An object of the present invention is to provide an endoscopic treatment tool and a water supply member capable of delivering fluid from a distal end.

According to one embodiment of the technology of the present disclosure, there is provided an endoscopic treatment tool comprising: a treatment tool main body of which a distal end part is configured to be opened and closed and which extends in a first direction; an operation member that is provided on a proximal end side of the treatment tool main body and is used for an opening and closing operation of the distal end part; and an accommodating member that has a tubular shape and that accommodates the treatment tool main body inside and has a through-hole allowing fluid to be supplied inside, in which the accommodating member and the treatment tool main body are configured to move relative to each other in the first direction so as to form a first state in which the treatment tool main body protrudes from a first opening provided on a distal end side of the accommodating member or a second state in which at least a part of a protrusion range of the treatment tool main body in the first state is retracted toward the proximal end side with respect to the first opening.

According to one embodiment of the technology of the present disclosure, there is provided an endoscopic treatment tool comprising: a treatment tool main body that extends in a first direction; an operation member that is provided on a proximal end side of the treatment tool main body and is used for operating the treatment tool main body; and an accommodating member that has a tubular shape and that accommodates the treatment tool main body inside and has a through-hole allowing fluid to be supplied inside, in which a position of the treatment tool main body with respect to the operation member is fixed, the accommodating member is configured to move in the first direction, and a first state in which the treatment tool main body protrudes from a first opening provided on a distal end side of the accommodating member and a second state in which at least a part of a protrusion range of the treatment tool main body in the first state is retracted toward the proximal end side with respect to the first opening is formed by movement of the accommodating member.

According to one embodiment of the technology of the present disclosure, there is provided an endoscopic treatment tool comprising: a treatment tool main body that extends in a first direction; an operation member that is provided on a proximal end side of the treatment tool main body and is used for operating the treatment tool main body; and an accommodating member that has a tubular shape and that accommodates the treatment tool main body inside and has a through-hole allowing fluid to be supplied inside, in which the accommodating member and the treatment tool main body are configured to move relative to each other in the first direction so as to form a first state in which the treatment tool main body protrudes from a first opening provided on a distal end side of the accommodating member or a second state in which at least a part of a protrusion range of the treatment tool main body in the first state is retracted toward the proximal end side with respect to the first opening, and the endoscopic treatment tool further comprises a first restricting portion that restricts a distal end of the treatment tool main body from moving a predetermined distance or more toward the proximal end side with respect to an opening edge of the first opening.

According to one embodiment of the technology of the present disclosure, there is provided a water supply member having a tubular shape into which a treatment tool main body extending in a first direction is capable of being inserted, the water supply member comprising: an outer side surface on which a through-hole is provided; and a first restricting portion that restricts a distal end of the inserted treatment tool main body from moving a predetermined distance or more toward a proximal end side with respect to an opening edge of a first opening provided on a distal end side of the water supply member.

According to the present invention, it is possible to provide an endoscopic treatment tool and a water supply member for an endoscope capable of delivering fluid from a distal end.

### BRIEF DESCRIPTION OF THE DRAWINGS

Fig. 1 is a schematic diagram showing a schematic configuration of a system including an endoscopic treatment tool 1 according to an embodiment of the present invention.
Fig. 2 is a cross-sectional view of a main part of the endoscopic treatment tool 1 in a protruding state.
Fig. 3 is an enlarged cross-sectional view of a vicinity of a proximal end of an accommodating member 2 and a vicinity of an operation member 4 of the endoscopic treatment tool 1 in a state during a transition from a retracted state to the protruding state.
Fig. 4 is an enlarged cross-sectional view of a vicinity of a distal end of the endoscopic treatment tool 1 in the retracted state.
Fig. 5 is an enlarged cross-sectional view of the vicinity of the distal end of the endoscopic treatment tool 1 in the protruding state.
Fig. 6 is an enlarged cross-sectional view showing a state in which a treatment tool distal end part 13 is opened in the protruding state shown in Fig. 5.
Fig. 7 is a cross-sectional view of a main part showing a first modification example of the endoscopic treatment tool 1.
Fig. 8 is a cross-sectional view of a main part showing a second modification example of the endoscopic treatment tool 1.
Fig. 9 is a cross-sectional view of a main part showing a third modification example of the endoscopic treatment tool 1.
Fig. 10 is a cross-sectional view taken along line A-A in Fig. 9.
Fig. 11 is a cross-sectional view of a main part showing a fourth modification example of the endoscopic treatment tool 1.
Fig. 12 is a cross-sectional view of a main part showing a fifth modification example of the endoscopic treatment tool 1.
Fig. 13 is a cross-sectional view of a main part showing a sixth modification example of the endoscopic treatment tool 1.
Fig. 14 is a cross-sectional view taken along line X2-X2 of Fig. 13.
Fig. 15 is a cross-sectional view of a main part showing a seventh modification example of the endoscopic treatment tool 1.
Fig. 16 is a cross-sectional view showing only a main part of a distal end part 2c of the accommodating member 2 in the endoscopic treatment tool 1 shown in Fig. 15.
Fig. 17 is a cross-sectional view of a main part showing an eighth modification example of the endoscopic treatment tool 1.
Fig. 18 is a cross-sectional view of a main part showing a ninth modification example of the endoscopic treatment tool 1.
Fig. 19 is a cross-sectional view of the endoscopic treatment tool 1 shown in Fig. 18 in a state in which the treatment tool distal end part is accommodated in the accommodating member.

### DESCRIPTION OF THE PREFERRED EMBODIMENTS

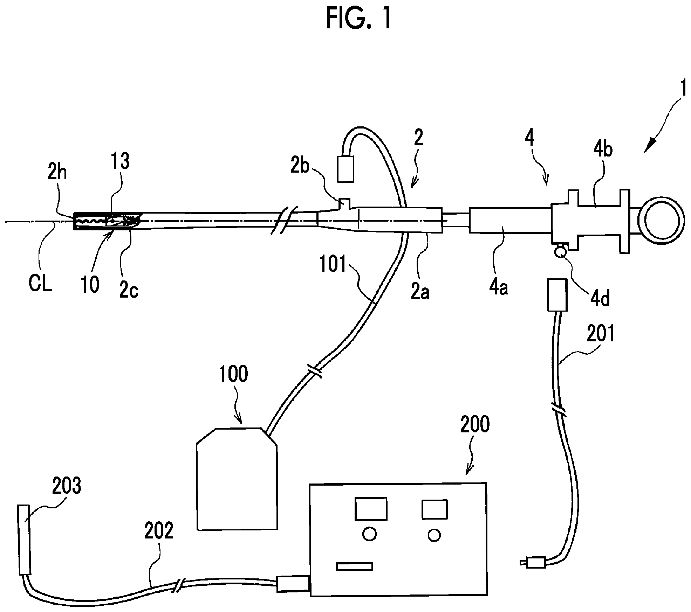

Fig. 1 is a schematic diagram showing a schematic configuration of a system including an endoscopic treatment tool 1 according to an embodiment of the present invention.

The system shown in Fig. 1 comprises the endoscopic treatment tool 1, a liquid supply device 100 that supplies a liquid such as water or local injection solutions, a supply pipe 101 that connects the endoscopic treatment tool 1 and the liquid supply device 100 to each other, a high-frequency power supply 200, a cable 201 that connects the endoscopic treatment tool 1 and the high-frequency power supply 200 to each other, a facing plate 203 to be attached to a treatment target (for example, a human body), and a cable 202 that connects the high-frequency power supply 200 and the facing plate 203 to each other.

The endoscopic treatment tool 1 is used by being inserted into a treatment tool insertion channel of an endoscope (not shown). Hereinafter, in the endoscopic treatment tool 1 in a state of being inserted into the treatment tool insertion channel, the outlet side of the treatment tool insertion channel is referred to as a distal end side, and the inlet side of the treatment tool insertion channel is referred to as a proximal end side.

The endoscopic treatment tool 1 comprises an elongated treatment tool main body 10 including a treatment tool distal end part 13 having an opening and closing structure (forceps structure) and provided at the distal end thereof, an operation member 4 that is provided on the proximal end side of the treatment tool main body 10 and that is used for an opening and closing operation of the treatment tool distal end part 13, and a tubular and elongated accommodating member 2 that accommodates the treatment tool main body 10.

The accommodating member 2 has a shape extending along an axis of the treatment tool insertion channel of the endoscope and has flexibility. The treatment tool main body 10 has a shape extending along the axis of the treatment tool insertion channel of the endoscope, and a portion excluding the treatment tool distal end part 13 has flexibility. Hereinafter, a direction along an axis CL of the accommodating member 2 is referred to as an axial direction.

In the endoscopic treatment tool 1, the position of the treatment tool main body 10 in the axial direction with respect to the operation member 4 is fixed (the treatment tool main body 10 and the operation member 4 are non-movable relative to each other in the axial direction). On the other hand, the accommodating member 2 is configured to move in the axial direction with respect to an assembly consisting of the operation member 4 and the treatment tool main body 10.

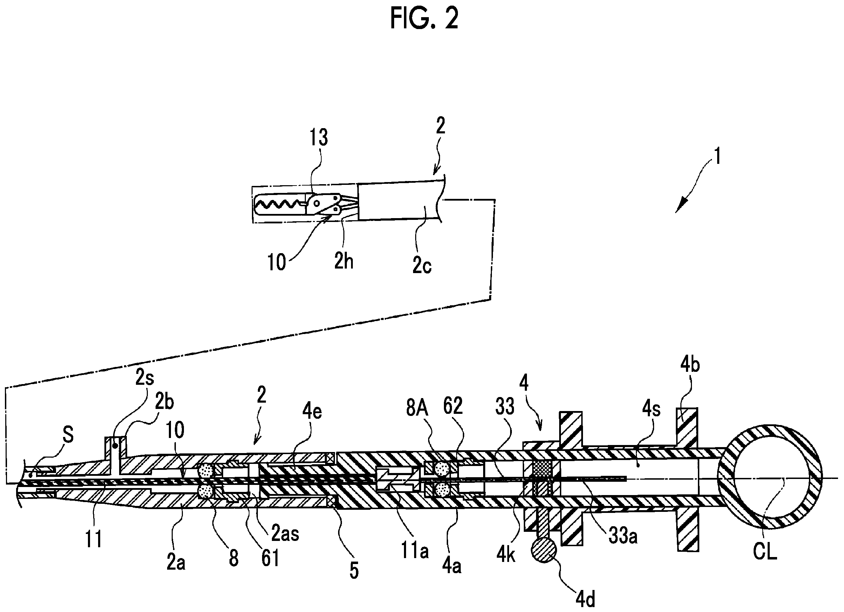

That is, the accommodating member 2 and the treatment tool main body 10 are configured to move relative to each other in the axial direction. More specifically, the accommodating member 2 and the treatment tool main body 10 are configured to move relative to each other in the axial direction such that a protruding state (see Fig. 2) in which the treatment tool distal end part 13 protrudes from a distal end opening 2h of the accommodating member 2 and a retracted state (see Fig. 1) in which the entire protrusion range of the treatment tool distal end part 13 from the distal end opening 2h in the protruding state is retracted toward the proximal end side with respect to the distal end opening 2h can be taken. In the protruding state, the treatment tool distal end part 13 protrudes from the distal end opening 2h to such an extent that the treatment tool distal end part 13 can be maximally opened. The protruding state constitutes a first state, and the retracted state constitutes a second state.

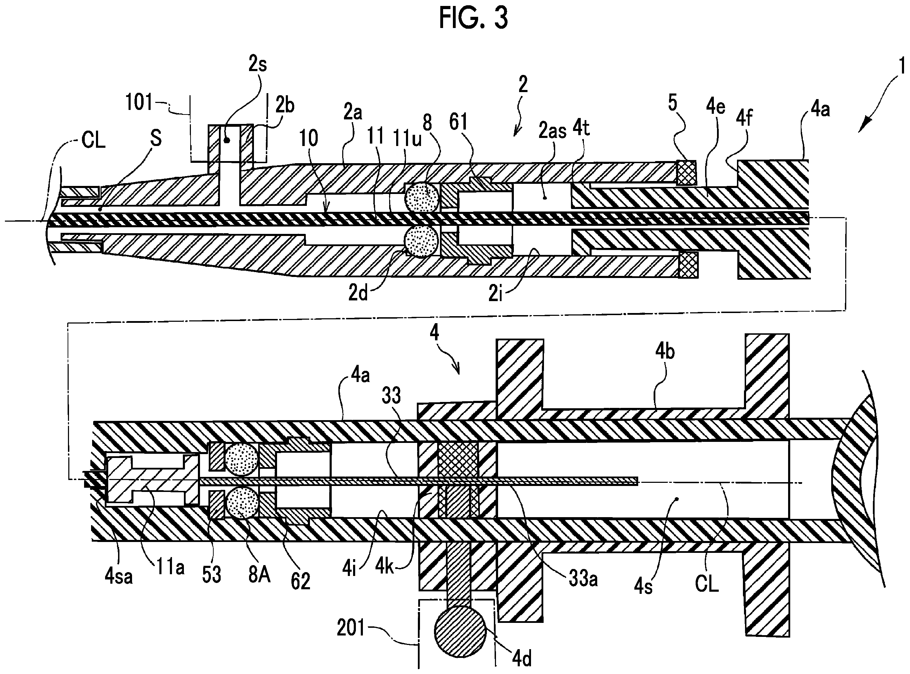

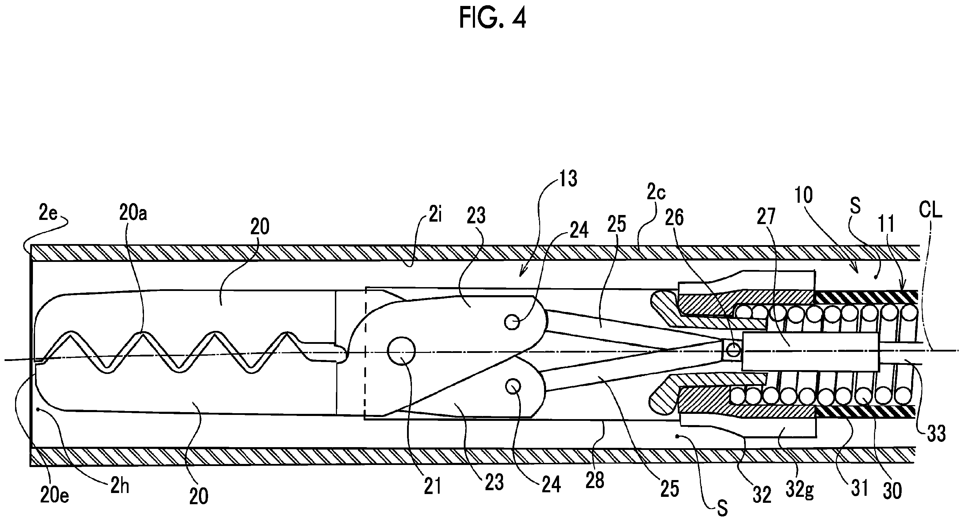

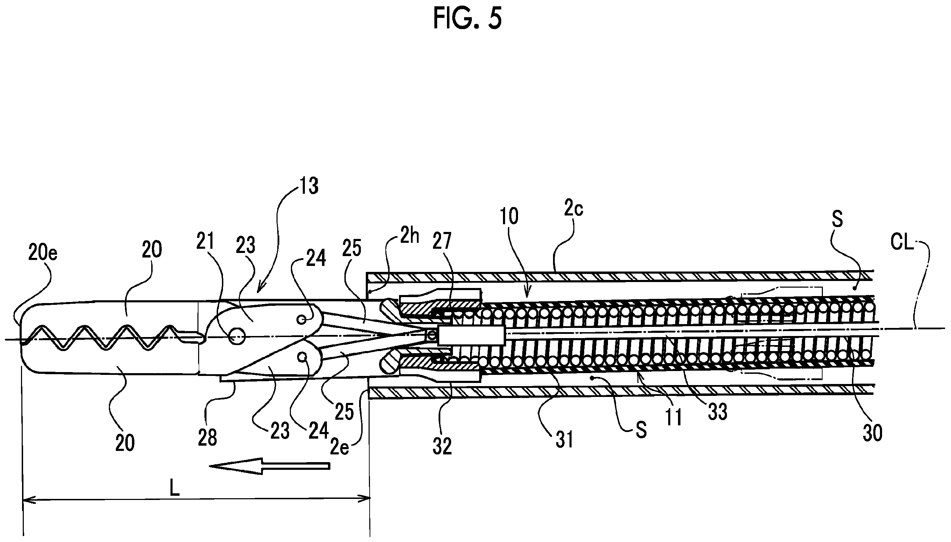

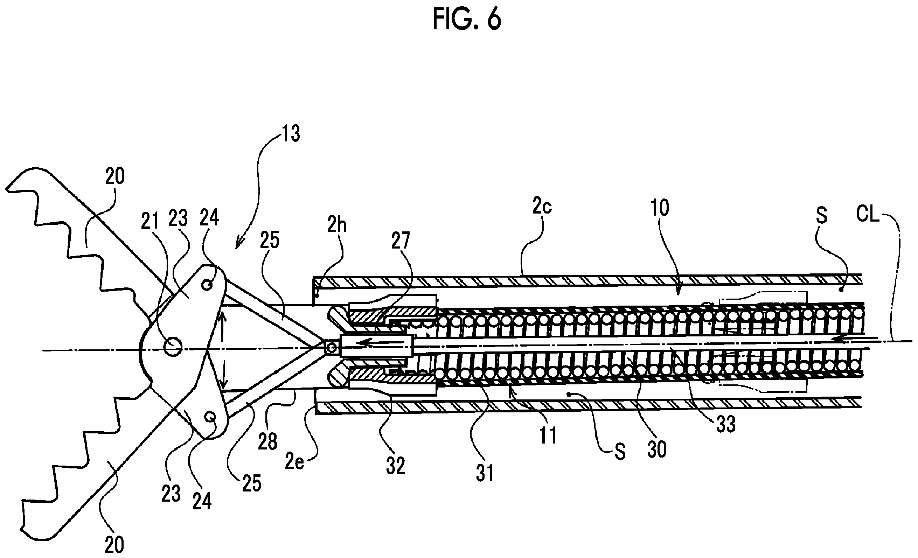

Fig. 2 is a cross-sectional view of a main part of the endoscopic treatment tool 1 in the protruding state. Fig. 3 is an enlarged cross-sectional view of the vicinity of the proximal end of the accommodating member 2 and the vicinity of the operation member 4 in a state during the transition from the retracted state to the protruding state. Fig. 4 is an enlarged cross-sectional view of the vicinity of the distal end of the endoscopic treatment tool 1 in the retracted state. Fig. 5 is an enlarged cross-sectional view of the vicinity of the distal end of the endoscopic treatment tool 1 in the protruding state. Fig. 6 is an enlarged cross-sectional view showing a state in which the treatment tool distal end part 13 is opened in the protruding state shown in Fig. 5.

In using the endoscopic treatment tool 1, in the retracted state shown in Fig. 1, an operation of pushing the operation member 4 into the accommodating member 2 or an operation of bringing the accommodating member 2 close to the operation member 4 is performed so that the position of the accommodating member 2 in the axial direction with respect to the treatment tool main body 10 is changed and switching from the retracted state to the protruding state is performed. Further, in the protruding state shown in Fig. 2, an operation of pulling out the operation member 4 from the accommodating member 2 or an operation of moving the accommodating member 2 away from the operation member 4 is performed so that switching from the protruding state to the retracted state is performed.

An operator such as a doctor who uses the endoscopic treatment tool 1 inserts the endoscopic treatment tool 1, which is in the retracted state shown in Fig. 1, into the distal end of the endoscope and protrudes the distal end (the distal end of the accommodating member 2) of the endoscopic treatment tool 1 from the distal end of the endoscope. In this state, for example, the doctor pulls a proximal end part 2a of the accommodating member 2 toward the proximal end side to protrude the treatment tool distal end part 13 from the distal end opening 2h, as shown in Fig. 2.

Then, the operator operates the operation member 4 to open and close the treatment tool distal end part 13 and uses the treatment tool distal end part 13 to resect a lesion part. After removing the lesion part, the doctor moves, for example, the accommodating member 2 to the distal end side and accommodates the entire treatment tool distal end part 13 in the accommodating member 2. After that, the doctor pulls out the endoscopic treatment tool 1 from the endoscope. Through the above procedures, treatments such as Endoscopic Submucosal Dissection (ESD) are performed.

As shown in Figs. 3 and 4, the treatment tool main body 10 comprises the treatment tool distal end part 13, a flexible cord 11 extending in the axial direction and stuck to the proximal end of the treatment tool distal end part 13, and a tubular cord fixing portion 11a that internally holds the proximal end of the flexible cord 11. The flexible cord 11 has a structure that is flexible in a radial direction (bending direction) of the cord, and is composed of a coil sleeve 30 consisting of a tightly wound coil and covered with an insulating tube 31, as shown in Fig. 4.

As shown in Fig. 4, the treatment tool distal end part 13 includes a sawtooth-shaped uneven portion 20a inside and comprises a pair of forceps pieces 20 that can be opened and closed about a support shaft 21, a link mechanism that opens and closes the pair of forceps pieces 20, an actuating member 27 that actuates the link mechanism, an attachment member 28 to which the support shaft 21 is attached, and a tubular distal end coupling member 32 stuck to the attachment member 28.

The link mechanism of the treatment tool distal end part 13 comprises a pair of link plate portions 23 consecutively provided at each forceps piece 20 and pivotally supported by the support shaft 21, a pair of link rods 25 coupled to each link plate portion 23 via a pivot shaft 24, and a pivot shaft 26 that connects each link rod 25 to the actuating member 27.

The distal end part of the coil sleeve 30 is stuck to the inside of the distal end coupling member 32. The actuating member 27 is provided inside the distal end part of the coil sleeve 30. The distal end of an operation wire 33, which will be described later, is stuck to the proximal end of the actuating member 27. The link mechanism is connected to the distal end of the actuating member 27. The distal end coupling member 32 shown in Fig. 4 includes a plurality of outer surface grooves 32g provided on an outer surface along the axial direction in order to improve the communication property of an internal space S of the accommodating member 2.

As shown in Fig. 3, the operation member 4 comprises a tubular main body part 4a coupled to the proximal end part 2a of the accommodating member 2, an operation element 4b supported by the main body part 4a, the operation wire 33 accommodated in the main body part 4a and having conductivity and flexibility, an annular plate 53 accommodated in the main body part 4a, a sealing member 8A, and a fixing member 62.

A distal end fitting portion 4e constituting the distal end part of the main body part 4a is configured to have a diameter smaller than that of other portions of the main body part 4a and is fitted into a proximal end side space 2as in the internal space S of the accommodating member 2. In an internal space 4s of the main body part 4a, the proximal end part of the treatment tool main body 10 (the proximal end part of the flexible cord 11 and the cord fixing portion 11a), the proximal end part of the operation wire 33, the annular plate 53, the sealing member 8A, and the fixing member 62 are accommodated.

The operation wire 33 is inserted into the inside of the flexible cord 11 from the proximal end side of the flexible cord 11 and reaches the actuating member 27 of the treatment tool distal end part 13. The operation wire 33 is electrically connected to the forceps piece 20 of the treatment tool distal end part 13 via the actuating member 27 and the link mechanism.

The cord fixing portion 11a accommodated in the internal space 4s of the main body part 4a is supported by an inner peripheral surface of the main body part 4a and is configured to rotate around the axis CL. The position of the cord fixing portion 11a in the main body part 4a in the axial direction is fixed. That is, as described above, the position of the treatment tool main body 10 in the axial direction with respect to the operation member 4 is fixed (the treatment tool main body 10 and the operation member 4 are non-movable relative to each other in the axial direction).

In the internal space 4s of the main body part 4a, the cord fixing portion 11a is provided on the proximal end side with respect to a distal end side end surface 4sa. In the internal space 4s, the annular plate 53 is provided next to the proximal end side of the cord fixing portion 11a, and the annular sealing member 8A including an O-ring or the like is provided next to the proximal end side of the annular plate 53. In the internal space 4s, the tubular fixing member 62 for pressing the sealing member 8A against the annular plate 53 to fix the sealing member 8A is provided next to the proximal end side of the sealing member 8A. The operation wire 33 is inserted into the annular plate 53, the sealing member 8A, and the fixing member 62 so as to be movable in the axial direction. The presence of the sealing member 8A makes it possible to prevent a liquid from leaking to an operation element 4b side even in a case in which the liquid has entered the internal space 4s from the opening on the distal end side of the main body part 4a.

The operation element 4b is configured to have, for example, a substantially H-shaped cross-section in appearance, and is supported by an outer peripheral surface of the proximal end part of the main body part 4a and is configured to slide in the axial direction with respect to the main body part 4a. The operation element 4b includes a slide piece portion 4k of which a part is incorporated in the main body part 4a. A proximal end part 33a of the operation wire 33 accommodated in the internal space 4s is stuck to the slide piece portion 4k. Therefore, by moving the operation element 4b in the axial direction, the operation wire 33 can be moved in the axial direction. In the protruding state shown in Fig. 5, in a case in which the operation wire 33 moves to the distal end side along the axial direction, as shown in Fig. 6, the actuating member 27 moves in the axial direction to actuate the link mechanism, and the forceps pieces 20 of the treatment tool distal end part 13 are opened.

As shown in Fig. 3, the slide piece portion 4k of the operation element 4b is provided with a connection terminal 4d to which the cable 201 is connected. The connection terminal 4d is provided to penetrate to the internal space 4s of the main body part 4a, is stuck to the proximal end part 33a of the operation wire 33, and is electrically connected to the operation wire 33. Therefore, a current can be supplied from the high-frequency power supply 200 to the treatment tool distal end part 13 via the cable 201, the connection terminal 4d, and the operation wire 33.

The endoscopic treatment tool 1 is configured as a monopolar high-frequency treatment tool in which a high-frequency current is applied to flow to the treatment tool distal end part 13 in a state in which the facing plate (patient plate) 203 connected to the high-frequency power supply 200 via the cable 202 are in contact with the surface of the subject's body skin. The endoscopic treatment tool 1 may be configured as a bipolar high-frequency treatment tool. In this case, the facing plate 203 is not required.

As shown in Figs. 2 and 3, the accommodating member 2 accommodates the treatment tool main body 10 in the internal space S. A connection protruding portion 2b to which the supply pipe 101 is connected is provided on the outer side surface of the proximal end part 2a of the accommodating member 2. A through-hole 2s that reaches the internal space S of the accommodating member 2 is formed in the connection protruding portion 2b. A communication path that communicates from the distal end opening 2h of the distal end part 2c to the through-hole 2s via the internal space S is formed inside the accommodating member 2.

By connecting the supply pipe 101 to the connection protruding portion 2b to deliver a liquid from the liquid supply device 100, the liquid can be ejected from the distal end opening 2h through this communication path. The inner diameter of the accommodating member 2 has a size such that a gap is formed between an inner peripheral surface 2i of the accommodating member 2 and the treatment tool main body 10 to allow fluid such as liquid to pass therethrough.

As shown in Fig. 3, the proximal end side space 2as formed in the proximal end part 2a of the accommodating member 2 is configured as a large cylindrical space in the internal space S. In the proximal end side space 2as, an inner surface stepped portion 2d having an inner diameter different from the inner peripheral surface 2i is formed. The proximal end side space 2as is provided with a sealing member 8 such as an O-ring disposed on the inner surface stepped portion 2d, and a fixing member 61 that presses the sealing member 8 against the inner surface stepped portion 2d to fix the sealing member 8.

As described above, the sealing member 8 is provided between the inner peripheral surface 2i of the proximal end part 2a of the accommodating member 2 and an outer peripheral surface 1 lu of the flexible cord 11. The presence of the sealing member 8 provided on the proximal end side with respect to the through-hole 2s in the accommodating member 2 makes it possible to prevent the liquid that has flowed into the internal space S from the through-hole 2s from moving to the proximal end side space 2as provided on the proximal end side with respect to the sealing member 8.

A restricting member 5 is stuck to a proximal end surface of the accommodating member 2. The restricting member 5 has, for example, an annular shape, and the inner peripheral edge thereof is located inward (on an axis CL side) of the inner peripheral surface 2i of the proximal end part 2a of the accommodating member 2 when viewed in the axial direction. The restricting member 5 is, for example, a member with high rigidity made of a metal, a resin, or the like. The distal end fitting portion 4e of the operation member 4 is inserted into the restricting member 5.

A main body distal end part 4t having an outer diameter substantially equal to the inner diameter of the proximal end side space 2as of the accommodating member 2 is provided at the distal end of the distal end fitting portion 4e. The main body distal end part 4t is in sliding contact with the inner peripheral surface 2i of the proximal end part 2a of the accommodating member 2. In this way, the accommodating member 2 is coupled to the main body part 4a so as to be slidable with respect to the main body part 4a of the operation member 4.

In a case in which the accommodating member 2 is slid toward the distal end side from the state shown in Fig. 3, the main body distal end part 4t and the restricting member 5 come into contact with each other to enter the retracted state shown in Fig. 4, and further movement of the accommodating member 2 toward the distal end side is restricted. The endoscopic treatment tool 1 is designed such that a distance in the axial direction between an opening edge 2e of the distal end opening 2h and a distal end 20e of the treatment tool distal end part 13 is a predetermined value in the retracted state shown in Fig. 4 in which the main body distal end part 4t and the restricting member 5 are in contact with each other. This predetermined value is preferably zero, but may be a value slightly larger than zero (for example, about several millimeters). It is preferable that this predetermined value is set to a value smaller than the maximum diameter of the treatment tool distal end part 13 in the closed state in a state viewed from the axial direction.

That is, in the retracted state shown in Fig. 4, it is preferable that the position of the opening edge 2e and the position of the distal end 20e match in the axial direction, but the position of the distal end 20e may slightly deviate to the proximal end side with respect to the position of the opening edge 2e. As described above, the restricting member 5 constitutes the first restricting portion that restricts the distal end 20e from moving a predetermined distance (the above predetermined value) or more toward the proximal end side with respect to the opening edge 2e.

In the retracted state shown in Fig. 4, the distal end 20e does not move the predetermined distance (the above predetermined value) or more toward the proximal end side with respect to the opening edge 2e, so that the liquid can be strongly discharged from the distal end opening 2h. In particular, in a case in which the position of the opening edge 2e and the position of the distal end 20e match in the retracted state, by taking a cross-section perpendicular to the axial direction at the position of the opening edge 2e, it is possible to minimize the cross-sectional area of the region through which the liquid passes, and the liquid can be most strongly discharged. In addition, by ensuring that the treatment tool distal end part 13 does not protrude outward beyond the opening edge 2e in the retracted state, the liquid discharged from the distal end opening 2h can be strongly discharged without being obstructed by the treatment tool distal end part 13. In this way, the above predetermined distance is set to such a small value that the liquid can be strongly discharged from the distal end opening 2h in the retracted state.

In the treatment such as ESD, a local injection solution such as physiological saline is injected into a lesion part to raise the submucosal layer, and the lesion resection is performed. With the endoscopic treatment tool 1, even in a case in which it is desired to add a local injection solution into a lesion part during a treatment such as ESD, the local injection solution can be added in a state in which the distal end of the endoscopic treatment tool 1 is in contact with the mucous membrane. As described above, since the operation of replacing the endoscopic treatment tool 1 during the treatment is not required, the treatment can be efficiently performed.

In a case in which the accommodating member 2 is slid toward the proximal end side from the state shown in Fig. 3, the restricting member 5 and a stepped end surface 4f of the operation member 4 come into contact with each other to enter the protruding state shown in Fig. 5, and further movement of the accommodating member 2 toward the proximal end side is restricted. A maximum amount of protrusion of the treatment tool distal end part 13 from the opening edge 2e, that is, a maximum distance L (see Fig. 5) between the distal end 20e and the opening edge 2e, is determined by the restricting member 5 and the stepped end surface 4f. As shown in Fig. 6, the maximum distance L need only be such a value that the forceps pieces 20 can be sufficiently opened.

As described above, in the endoscopic treatment tool 1, the restricting member 5 and the stepped end surface 4f constitute a second restricting portion that restricts the maximum distance L between the distal end 20e and the opening edge 2e in the protruding state to a predetermined value. With this second restricting portion, the treatment tool distal end part 13 can be held by protruding at an appropriate distance from the accommodating member 2 in the protruding state. Therefore, the operation of the treatment tool distal end part 13 can be stably performed.

In the endoscopic treatment tool 1, the entire protrusion range (a range indicated by the maximum distance L shown in Fig. 5) of the treatment tool main body 10 protruding from the distal end opening 2h in the protruding state is retracted toward the proximal end side from the distal end opening 2h in the retracted state shown in Fig. 4. However, in the retracted state shown in Fig. 4, a configuration may be employed in which a part of the protrusion range is retracted from the distal end opening 2h to the proximal end side (in other words, a configuration in which a part of the above protrusion range protrudes from the distal end opening 2h).

For example, in a configuration in which the main body distal end part 4t shown in Fig. 3 is slightly moved to the right side in the drawing, the forceps pieces 20 enter a state of partially protruding from the distal end opening 2h in the retracted state. However, even in this state, the liquid can be more strongly discharged than the liquid is discharged from the distal end opening 2h in the protruding state shown in Fig. 5.

In the endoscopic treatment tool 1, the restricting member 5 is made of a metal, a resin, or the like, but the restricting member 5 may be made of, for example, an elastic body such as rubber. By doing so, the accommodating member 2 is attachable to and detachable from the assembly of the treatment tool main body 10 and the operation member 4 while positioning the distal end 20e in the protruding state and the retracted state. For example, in a case in which the accommodating member 2 is further moved to the distal end side in a state in which the restricting member 5 is in contact with the main body distal end part 4t, the restricting member 5 can move to the distal end side by passing over the main body distal end part 4t by the elasticity of the restricting member 5. In a case in which the restricting member 5 passes over the main body distal end part 4t, the accommodating member 2 can move to the distal end side without limitation. As a result, the accommodating member 2 can be removed from the treatment tool main body 10 and the operation member 4.

In a case in which the accommodating member 2 is configured to be attached and detached as described above, the same structure as the endoscopic treatment tool 1 can be obtained only by mounting the accommodating member 2 on the existing endoscopic treatment tool (which is close to the assembly). Therefore, it is possible to reduce the manufacturing cost of the endoscopic treatment tool 1. In addition, by attaching and detaching the accommodating member 2 in accordance with the purpose of the treatment, various ways of use depending on the treatment are possible.

The endoscopic treatment tool 1 is configured such that the treatment tool main body 10 and the operation member 4 are non-movable relative to each other in the axial direction, but a configuration may be employed as a modification example in which, for example, the treatment tool main body 10 is configured to move to the operation member 4 in the axial direction. In a case of employing this configuration, for example, a mechanism for moving the treatment tool main body 10 in the axial direction is provided inside the main body part 4a of the operation member 4, and the operation member 4 need only be operated to move the treatment tool main body 10 in the axial direction. In addition, by sticking the operation member 4 and the accommodating member 2 to each other, the operation member 4 and the accommodating member 2 need only be made non-movable relative to each other in the axial direction. Even with this configuration, it is possible to switch between the retracted state shown in Fig. 4 and the protruding state shown in Fig. 5 by operating the operation member 4. In the retracted state, the liquid can be strongly discharged.

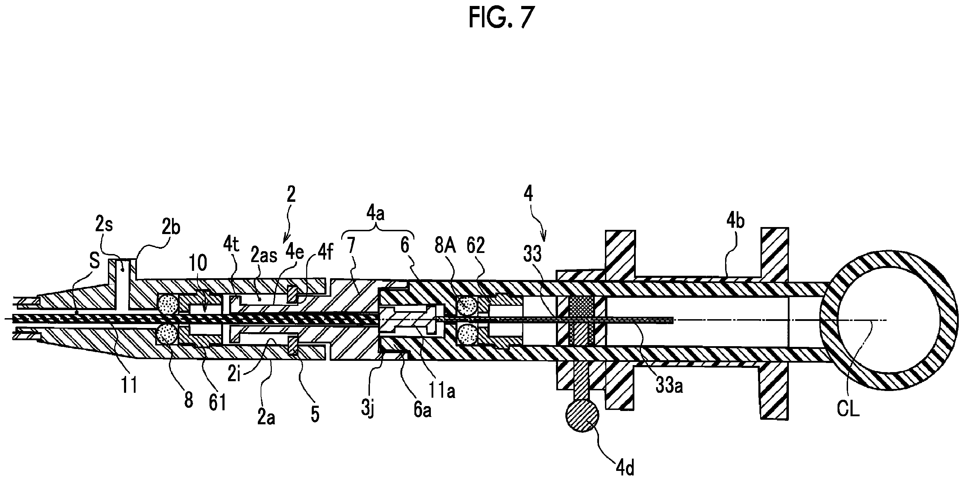

Fig. 7 is a cross-sectional view of a main part showing a first modification example of the endoscopic treatment tool 1. Regarding the description of the reference numerals shown in Fig. 7, the same configurations as those shown in Fig. 2 are designated by the same reference numerals, and the description thereof will be omitted.

In the endoscopic treatment tool 1 shown in Fig. 7, the main body part 4a of the operation member 4 in the endoscopic treatment tool 1 shown in Fig. 2 is composed of two members, that is, a main body part 7 on the distal end side and a main body part 6 on the proximal end side. The main body part 7 has the same structure as the distal end part of the main body part 4a shown in Fig. 2 and includes the distal end fitting portion 4e, the main body distal end part 4t, and the stepped end surface 4f. In addition, the main body part 7 includes a coupling recessed portion 3j in the proximal end surface.

The main body part 6 has substantially the same structure as a portion other than the proximal end part of the main body part 4a shown in Fig. 2, but is different in that a coupling projecting portion 6a is provided at the distal end thereof. The coupling projecting portion 6a of the main body part 6 is fitted to the coupling recessed portion 3j of the main body part 7.

In the present modification example, the restricting member 5 is provided on the inner peripheral surface 2i of the proximal end part 2a of the accommodating member 2 and comes into contact with the main body distal end part 4t and the stepped end surface 4f of the main body part 7 to restrict the position of the distal end 20e of the treatment tool main body 10. In addition, the cord fixing portion 11a is incorporated in and attached to an accommodation space formed in a fitting portion between the coupling recessed portion 3j and the coupling projecting portion 6a.

With the endoscopic treatment tool 1 shown in Fig. 7, since the cord fixing portion 11a is provided in the accommodation space formed in the fitting portion between the coupling recessed portion 3j and the coupling projecting portion 6a, the cord fixing portion 11a is easily incorporated and the manufacturing cost can be reduced.

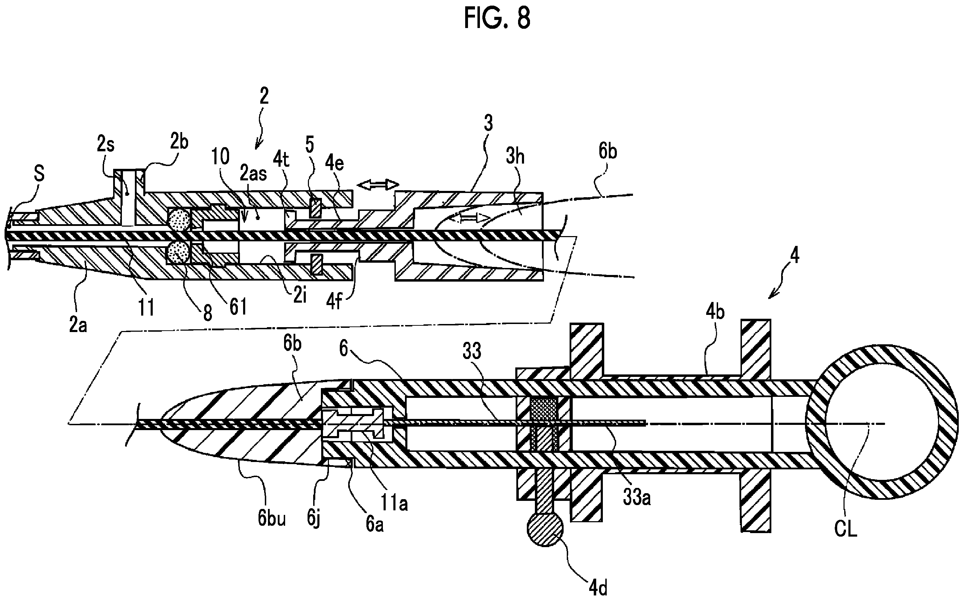

Fig. 8 is a cross-sectional view of a main part showing a second modification example of the endoscopic treatment tool 1. Regarding the description of the reference numerals shown in Fig. 8, the same configurations as those described above are designated by the same reference numerals, and the description thereof will be omitted.

In the endoscopic treatment tool 1 shown in Fig. 8, the main body part 7 in Fig. 7 is changed to a coupling member 3, and a main body part 6b is further added.

The coupling member 3 has the same structure as the distal end part of the main body part 4a shown in Fig. 2 and includes the distal end fitting portion 4e, the main body distal end part 4t, and the stepped end surface 4f. In addition, a relatively deep opening 3h is provided on the proximal end surface of the coupling member 3.

The main body part 6b is configured with a curved surface such that an outer surface 6bu thereof gradually decreases in diameter toward the distal end and bulges outward in a side view. The main body part 6b includes the coupling recessed portion 6j that is fitted to the coupling projecting portion 6a of the main body part 6 in the proximal end surface. The main body part 6b and the main body part 6 are stuck to each other through fitting between the coupling projecting portion 6a and the coupling recessed portion 6j. The main body part 6b is inserted and held so as to be pushed into the opening 3h of the coupling member 3. The coupling member 3 is attachable to and detachable from the main body part 6b.

In the present modification example, at least one of the coupling member 3 or the main body part 6b is made of, for example, an elastic material such as silicon rubber. As a result, the insertion depth of the main body part 6b can be freely changed with respect to the opening 3h. By adjusting the insertion depth, the protrusion range of the treatment tool main body 10 in the protruding state can be made larger or smaller than the above maximum distance L. The amount of protrusion can be adjusted in the protruding state, so that it is possible to perform treatment with an appropriate amount of protrusion in conformity with the preference of the user, and it is possible to perform good treatment.

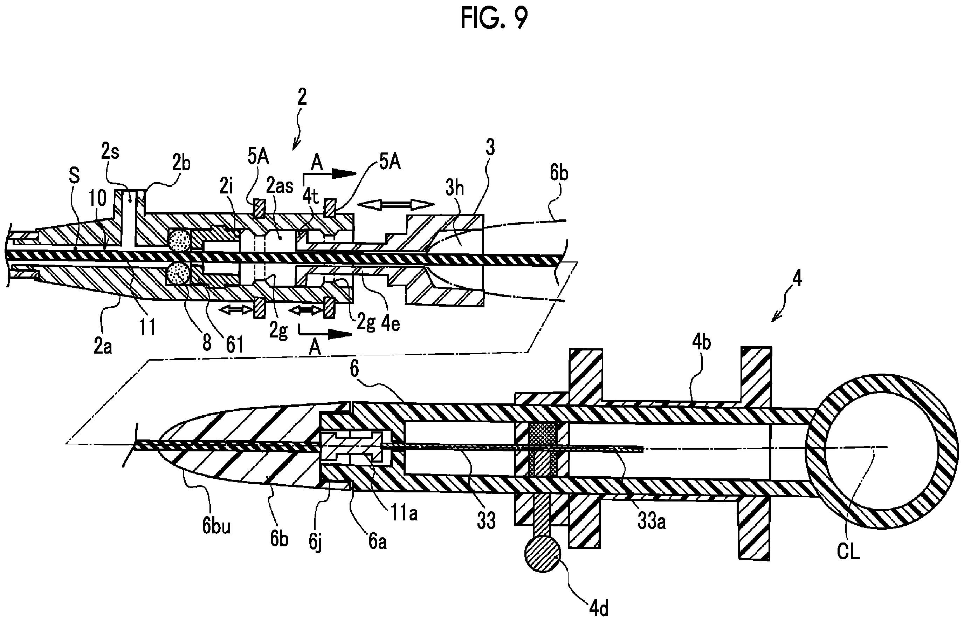

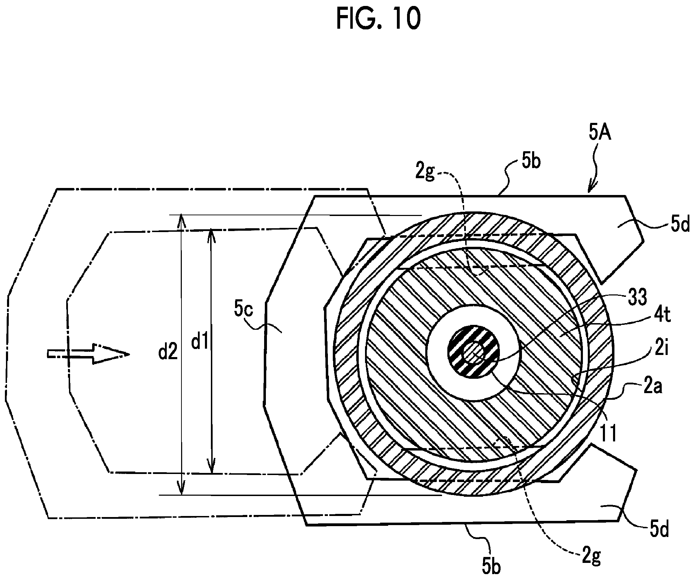

Fig. 9 is a cross-sectional view of a main part showing a third modification example of the endoscopic treatment tool 1. Fig. 10 is a cross-sectional view taken along line A-A in Fig. 9. Regarding the description of the reference numerals shown in Figs. 9 and 10, the same configurations as those described above are designated by the same reference numerals, and the description thereof will be omitted.

The endoscopic treatment tool 1 shown in Fig. 9 has a configuration in which the restricting member 5 is changed to two restricting members 5A in the endoscopic treatment tool 1 shown in Fig. 8. However, in the case of the present modification example, the depth of the opening 3h of the coupling member 3 is configured to be shallower than that of the configuration of Fig. 8, and a configuration in which the adjustment range of the insertion depth of the main body part 6b with respect to the coupling member 3 is small or a configuration in which the adjustment range does not exist is employed.

The two restricting members 5A are mounted on the outer surface of the proximal end part 2a of the accommodating member 2 that is elastically deformable at positions spaced apart from each other in the axial direction. As shown in Fig. 10, in a front view (in a state viewed from the axial direction), for example, the restricting member 5A is a substantially C-shaped member composed of two clamping pieces 5b extending substantially parallel to each other in the left-right direction in Fig. 10 and a coupling piece 5c coupling the two clamping pieces 5b at respective one end sides. A distal end locking piece 5d is provided on the other end side (distal end side) of each of the two clamping pieces 5b.

A distance dl between the two clamping pieces 5b is configured to be smaller than an external dimension d2 of the proximal end part 2a. Therefore, in a case in which the restricting member 5A is mounted on the outer surface of the proximal end part 2a, a projecting portion 2g is formed on the inner peripheral surface 2i of the accommodating member 2.

When mounting the restricting member 5A, as shown in Fig. 10, the restricting member 5A is mounted so as to be pushed from the radial direction of the proximal end part 2a. In the pushed restricting member 5A, the clamping pieces 5b press the outer surface of the proximal end part 2a to deform the outer surface inward. As a result, the projecting portion 2g is formed on the inner peripheral surface 2i of the proximal end part 2a. In the case of the present modification example, the two restricting members 5A are provided at a predetermined interval in the axial direction. Therefore, the main body distal end part 4t is movable between the two projecting portions 2g formed in the axial direction.

In the endoscopic treatment tool 1 shown in Fig. 9, in a case in which the main body distal end part 4t comes into contact with the projecting portion 2g formed by the restricting member 5A on the right side in the drawing, the retracted state described above so far is formed. In addition, in a case in which the main body distal end part 4t comes into contact with the projecting portion 2g formed by the restricting member 5A on the left side in the drawing, the protruding state described above so far is formed.

In the endoscopic treatment tool 1 shown in Fig. 9, the restricting member 5A on the right side constitutes the first restricting portion that restricts the distal end 20e from moving a predetermined distance (the above predetermined value) or more toward the proximal end side with respect to the opening edge 2e in the retracted state. Further, the restricting member 5A on the left side constitutes the second restricting portion that restricts the maximum distance L between the distal end 20e and the opening edge 2e to a predetermined value in the protruding state.

The restricting member 5A is attached to and detached from the accommodating member 2 so that the mounting position can be arbitrarily changed. In addition to the above, for example, the restricting member 5A can also be moved in the axial direction while being mounted on the proximal end part 2a. By changing the position of the restricting member 5A in the axial direction, it is possible to adjust the amount of protrusion of the treatment tool distal end part 13 in the protruding state.

In the endoscopic treatment tool 1 shown in Fig. 9, the coupling member 3 and the main body part 6b may be non-attachable and non-detachable by being stuck to each other. Even in such a manner, in the endoscopic treatment tool 1 shown in Fig. 9, since the restricting member 5A is removed from the accommodating member 2 so that the accommodating member 2 can be removed from the coupling member 3, the accommodating member 2 is attachable and detachable.

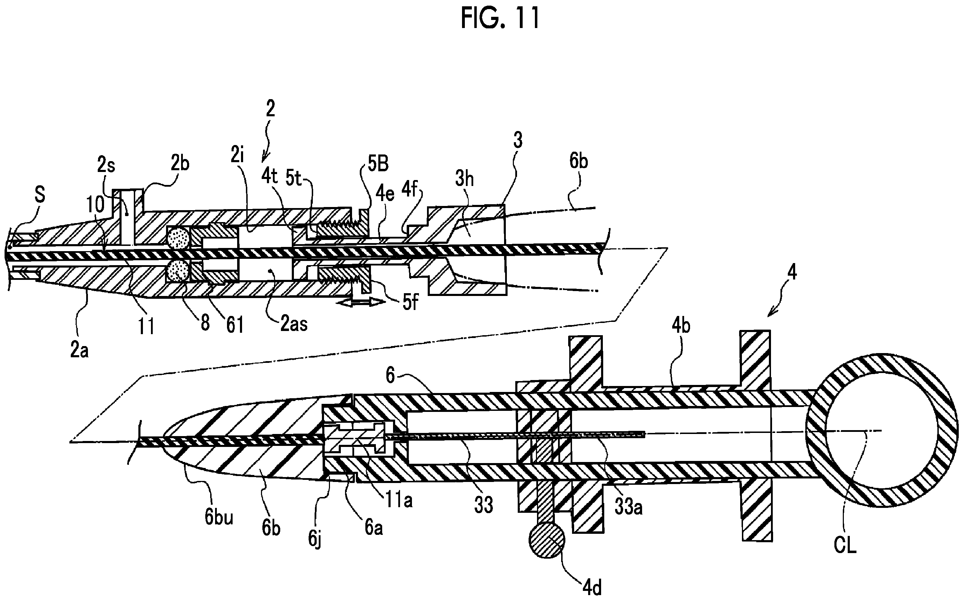

Fig. 11 is a cross-sectional view of a main part showing a fourth modification example of the endoscopic treatment tool 1. Regarding the description of the reference numerals shown in Fig. 11, the same configurations as those described above are designated by the same reference numerals, and the description thereof will be omitted.

The endoscopic treatment tool 1 shown in Fig. 11 is the same as the endoscopic treatment tool 1 shown in Fig. 9 except that the restricting member 5A is changed to a restricting member 5B. The restricting member 5B is configured to be screwed into the inner peripheral surface 2i of the proximal end part 2a of the accommodating member 2. The restricting member 5B is, for example, a substantially cylindrical member including a flange portion 5f on one end side (right side in the drawing). A male screw provided on the cylindrical outer peripheral surface of the restricting member 5B is screwed to a female screw of the inner peripheral surface 2i of the accommodating member 2 in a state in which the flange portion 5f is exposed to the proximal end side. Further, in the restricting member 5B, the main body distal end part 4t comes into contact with a distal end surface 5t thereof so that the distal end 20e is positioned in the retracted state. In addition, the stepped end surface 4f comes into contact with the flange portion 5f, so that the distal end 20e is positioned in the protruding state.

The restricting member 5B is movable in the axial direction by rotating with respect to the accommodating member 2, and the position of attachment thereof is adjustable. By adjusting the position of the restricting member 5B in the axial direction, it is possible to adjust the amount of protrusion of the treatment tool distal end part 13 in the protruding state, which allows for ways of use depending on the preference of the user.

In the endoscopic treatment tool 1 shown in Fig. 11, the restricting member 5B constitutes the first restricting portion that restricts the distal end 20e from moving a predetermined distance (the above predetermined value) or more toward the proximal end side with respect to the opening edge 2e in the retracted state. Further, the restricting member 5B and the stepped end surface 4f constitute the second restricting portion that restricts the maximum distance L between the distal end 20e and the opening edge 2e to a predetermined value in the protruding state.

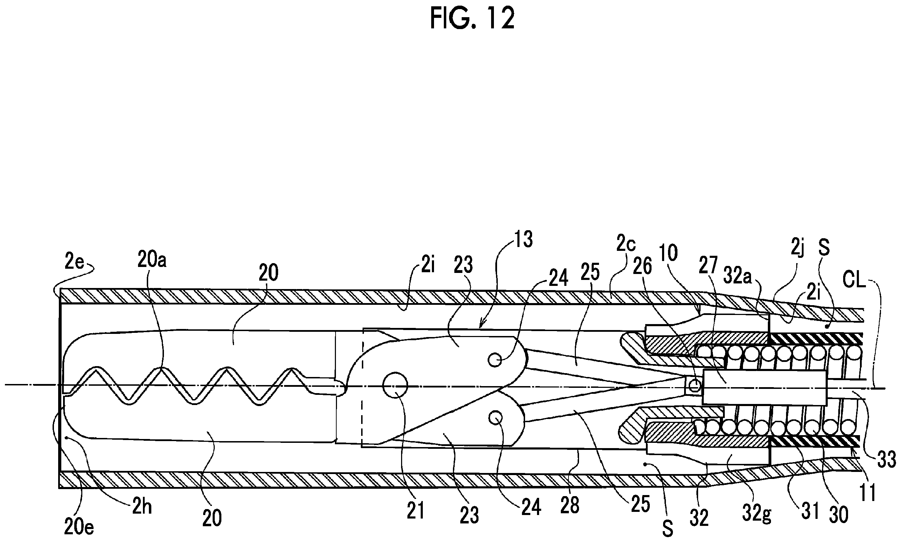

In the description so far, the distal end 20e is positioned in the retracted state by the restricting member 5, the restricting member 5A, the restricting member 5B, and the like provided in the proximal end part 2a of the accommodating member 2. However, a structure for positioning the distal end 20e in the retracted state can also be provided at the distal end part 2c of the accommodating member 2. Hereinafter, a configuration example in which the distal end 20e is positioned at the distal end part 2c will be described with reference to Figs. 12 to 17.

Fig. 12 is a cross-sectional view of a main part showing a fifth modification example of the endoscopic treatment tool 1. Regarding the description of the reference numerals shown in Fig. 12, the same configurations as those described above are designated by the same reference numerals, and the description thereof will be omitted.

In the endoscopic treatment tool 1 shown in Fig. 12, the shape of the distal end part 2c of the accommodating member 2 of the endoscopic treatment tool 1 shown in Fig. 4 is changed. Specifically, the distal end part 2c of the accommodating member 2 is provided with a diameter-reduced portion 2j whose inner diameter gradually decreases toward the proximal end side.

In the retracted state shown in Fig. 12, a proximal-end end surface 32a (the corner portion of the proximal-end end surface 32a) of the distal end coupling member 32 comes into contact with the inner peripheral surface 2i of the diameter-reduced portion 2j, so that further movement of the treatment tool distal end part 13 toward the proximal end side is restricted. In the endoscopic treatment tool 1 shown in Fig. 12, the diameter-reduced portion 2j constitutes the first restricting portion that restricts the distal end 20e from moving a predetermined distance or more toward the proximal end side with respect to the opening edge 2e in the retracted state.

Since the distal end coupling member 32 is provided with the outer surface grooves 32g, the communication path leading to the distal end opening 2h, the internal space S, and the through-hole 2s is maintained even in a state in which the corner portion of the proximal-end end surface 32a is in contact with the inner peripheral surface 2i.

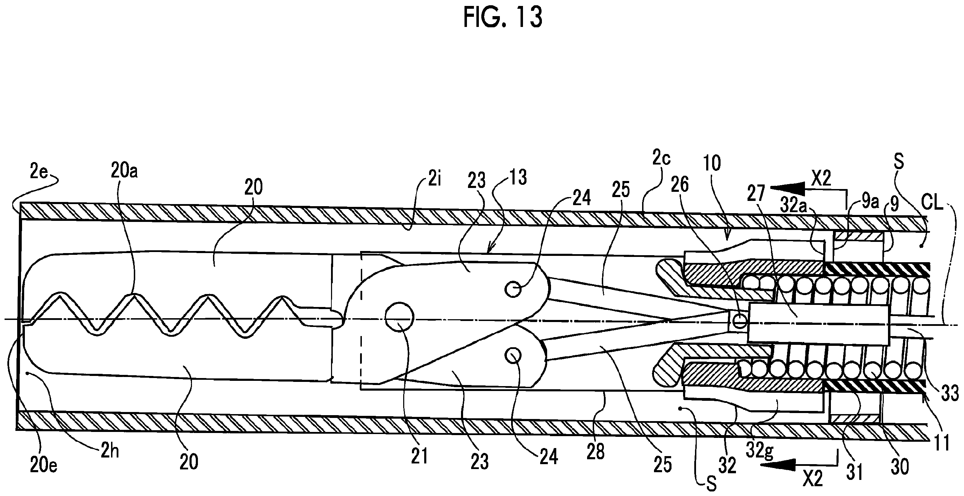

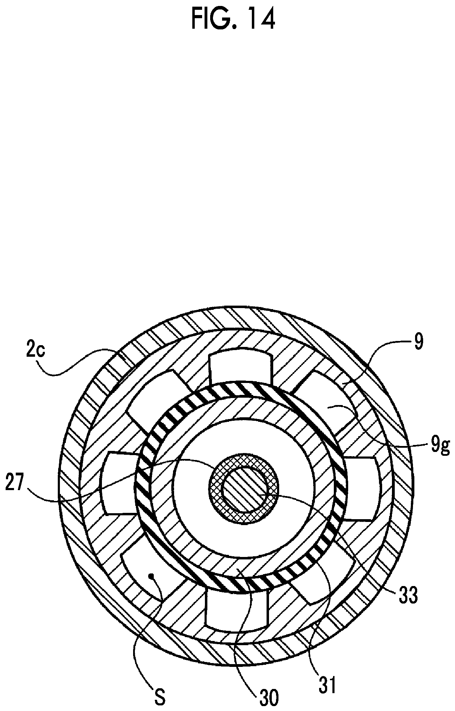

Fig. 13 is a cross-sectional view of a main part showing a sixth modification example of the endoscopic treatment tool 1. Fig. 14 is a cross-sectional view taken along line X2-X2 of Fig. 13. Regarding the description of the reference numerals shown in Fig. 13, the same configurations as those described above are designated by the same reference numerals, and the description thereof will be omitted.

In the endoscopic treatment tool 1 shown in Fig. 13, a configuration is employed in which a ring member 9 made of, for example, a metal is stuck to the inner peripheral surface 2i of the distal end part 2c of the accommodating member 2 of the endoscopic treatment tool 1 shown in Fig. 4. The ring member 9 is provided so as to be able to come into contact with the proximal-end end surface 32a of the distal end coupling member 32 on the proximal end side with respect to the distal end coupling member 32.

As shown in Fig. 14, a plurality of inner surface grooves 9g extending along the axial direction are provided on the inner peripheral side of the ring member 9. Even in a state in which the proximal-end end surface 32a of the distal end coupling member 32 is in contact with an end surface 9a on the distal end side of the ring member 9, the internal space S is not blocked because of the inner surface grooves 9g.

In the retracted state shown in Fig. 13, the proximal-end end surface 32a of the distal end coupling member 32 comes into contact with the end surface 9a of the ring member 9, so that further movement of the treatment tool distal end part 13 toward the proximal end side is restricted. In the endoscopic treatment tool 1 shown in Fig. 13, the ring member 9 constitutes the first restricting portion that restricts the distal end 20e from moving a predetermined distance or more toward the proximal end side with respect to the opening edge 2e in the retracted state.

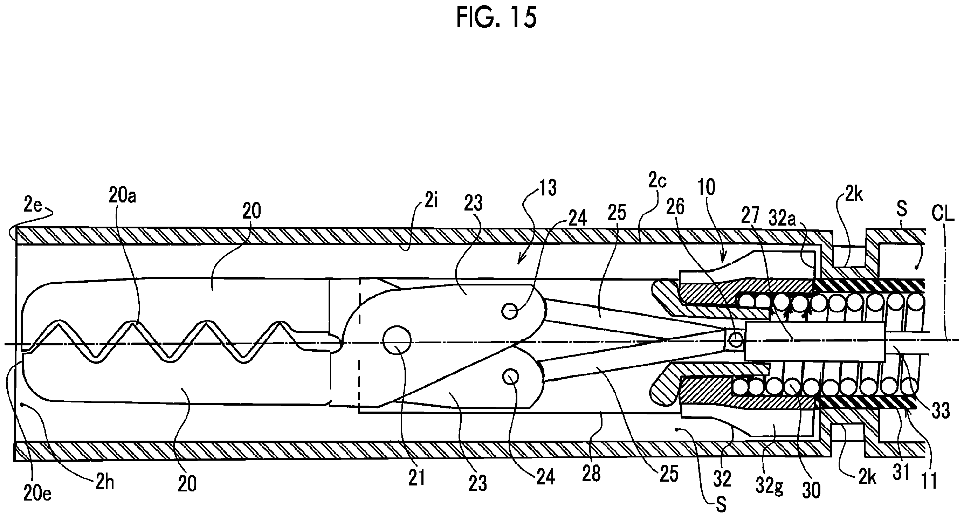

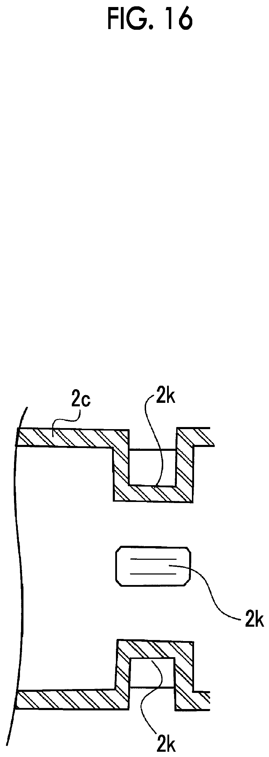

Fig. 15 is a cross-sectional view of a main part showing a seventh modification example of the endoscopic treatment tool 1. Fig. 16 is a cross-sectional view showing only a main part of the distal end part 2c of the accommodating member 2 in the endoscopic treatment tool 1 shown in Fig. 15. Regarding the description of the reference numerals shown in Fig. 15, the same configurations as those described above are designated by the same reference numerals, and the description thereof will be omitted.

The endoscopic treatment tool 1 shown in Fig. 15 has a configuration in which a plurality of outer surface recessed portions 2k are added to the outer surface of the distal end part 2c of the accommodating member 2 of the endoscopic treatment tool 1 shown in Fig. 4. As shown in Fig. 16, the plurality of outer surface recessed portions 2k are provided at predetermined intervals along the circumferential direction of the outer peripheral surface of the accommodating member 2. By forming the outer surface recessed portions 2k, projecting portions are formed on the inner peripheral surface 2i of the accommodating member 2.

In the retracted state shown in Fig. 15, the proximal-end end surface 32a of the distal end coupling member 32 comes into contact with the projecting portions of the inner peripheral surface 2i, so that further movement of the treatment tool distal end part 13 toward the proximal end side is restricted. In the endoscopic treatment tool 1 shown in Fig. 15, the outer surface recessed portions 2k constitute the first restricting portion that restricts the distal end 20e from moving a predetermined distance or more toward the proximal end side with respect to the opening edge 2e in the retracted state.

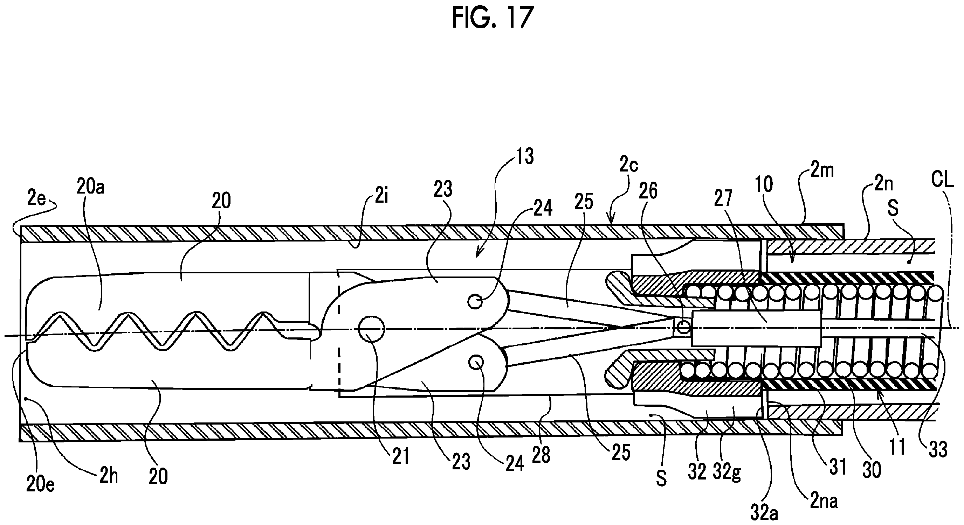

Fig. 17 is a cross-sectional view of a main part showing an eighth modification example of the endoscopic treatment tool 1. Regarding the description of the reference numerals shown in Fig. 17, the same configurations as those described above are designated by the same reference numerals, and the description thereof will be omitted.

In the endoscopic treatment tool 1 shown in Fig. 17, the distal end part 2c of the accommodating member 2 of the endoscopic treatment tool 1 shown in Fig. 4 is composed of a tubular large-diameter member 2m and a tubular small-diameter member 2n having a smaller diameter than the large-diameter member 2m. The small-diameter member 2n is fitted into the large-diameter member 2m from an opening of the large-diameter member 2m on the proximal end side and is stuck to the large-diameter member 2m by, for example, welding.

In the retracted state shown in Fig. 17, the proximal-end end surface 32a of the distal end coupling member 32 comes into contact with an end surface 2na of the small-diameter member 2n on the distal end side, so that further movement of the treatment tool distal end part 13 toward the proximal end side is restricted. Also in the endoscopic treatment tool 1 shown in Fig. 17, since the outer surface grooves 32g are formed on the distal end coupling member 32, the internal space S is not blocked in a contact portion between the end surface 2na of the small-diameter member 2n and the proximal-end end surface 32a of the distal end coupling member 32.

In the endoscopic treatment tool 1 shown in Fig. 17, the distal end of the small-diameter member 2n constitutes the first restricting portion that restricts the distal end 20e from moving a predetermined distance or more toward the proximal end side with respect to the opening edge 2e in the retracted state.

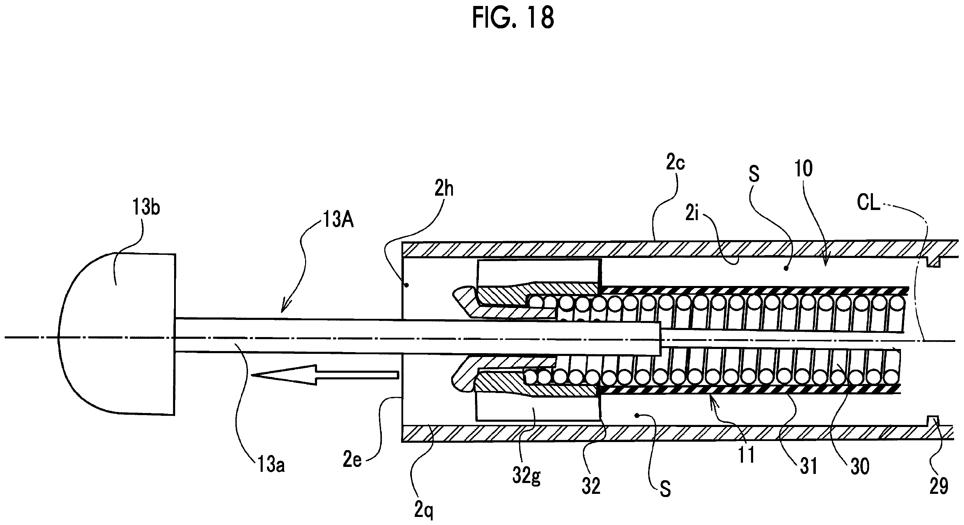

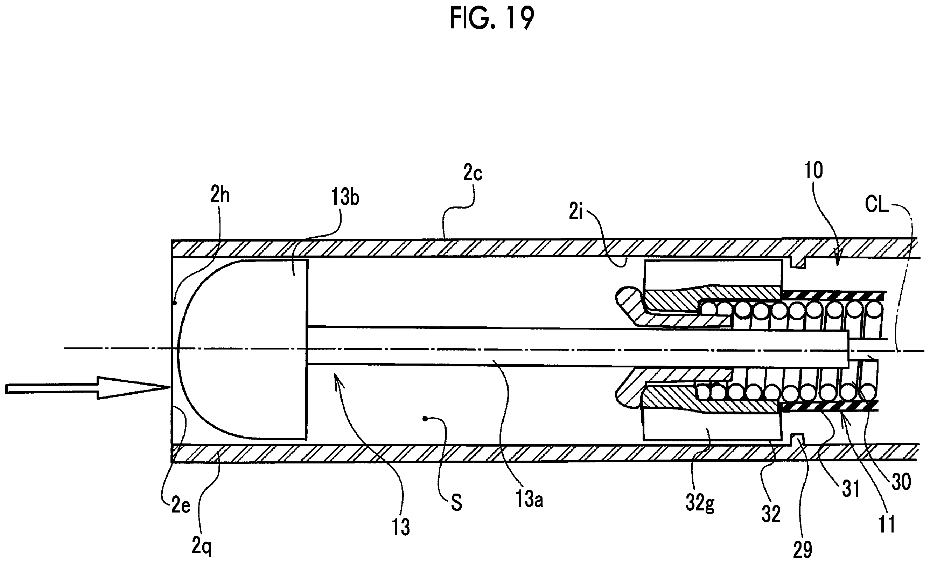

Figs. 18 and 19 are each a cross-sectional view of a main part showing a ninth modification example of the endoscopic treatment tool 1. Fig. 18 is a cross-sectional view of a state in which the treatment tool distal end part protrudes from the distal end opening of the accommodating member. Fig. 19 is a cross-sectional view of a state in which the treatment tool distal end part is accommodated in the accommodating member. Regarding the description of the reference numerals shown in Figs. 18 and 19, the same configurations as those described above are designated by the same reference numerals, and the description thereof will be omitted.

The endoscopic treatment tool 1 shown in Fig. 18 has a configuration in which the treatment tool distal end part 13 and the actuating member 27 in the endoscopic treatment tool 1 shown in Fig. 4 are changed to a treatment tool distal end part 13A. Further, in the endoscopic treatment tool 1 shown in Fig. 18, the treatment tool main body 10 is configured to move in the axial direction with respect to the operation member 4. Specifically, a mechanism for moving the treatment tool main body 10 in the axial direction is provided inside the main body part 4a of the operation member 4 (not shown), and the treatment tool main body 10 can be moved in the axial direction by operating the operation member 4. In addition, the operation member 4 and the accommodating member 2 are stuck to each other, and the operation member 4 and the accommodating member 2 are non-movable relative to each other in the axial direction.

The treatment tool distal end part 13A is configured as a high-frequency knife capable of performing incision or the like in a lesion part. The treatment tool distal end part 13A includes a rod-shaped trunk portion 13a that protrudes thinly, and a leading distal end portion 13b having a large diameter and located at a distal end of the trunk portion 13a. The trunk portion 13a is composed of an electrode (conductive member). The leading distal end portion 13b is composed of an insulating chip. The leading distal end portion 13b is provided with an electrode pair with an electrically insulating member appropriately disposed. The leading distal end portion 13b is configured to have the largest diameter in the treatment tool distal end part 13A when viewed from the axial direction (even when viewed from a side view as shown in the drawing), and is configured to have a size that fits within the accommodating member 2. The proximal end of the trunk portion 13a is stuck to the distal end coupling member 32 stuck to the distal end of the treatment tool main body 10. Therefore, in a case in which the treatment tool main body 10 moves in the axial direction through the operation of the operation member 4, the treatment tool distal end part 13A moves in the axial direction in conjunction with the movement.

Further, in the endoscopic treatment tool 1 shown in Fig. 18, for example, an annular protrusion 29 is provided on the inner peripheral surface 2i of the distal end part 2c of the accommodating member 2. By moving the treatment tool main body 10 to the distal end side, as shown in Fig. 18, the protruding state in which the treatment tool distal end part 13A protrudes from the distal end opening 2h is formed. In a case in which the treatment tool main body 10 is moved from the state shown in Fig. 18 to the proximal end side, the retracted state in which the entire treatment tool distal end part 13A that has been located outside the distal end opening 2h in the protruding state is accommodated inside the accommodating member 2 is formed as shown in Fig. 19.

In this retracted state, the distal end coupling member 32 comes into contact with the protrusion 29, so that further movement of the treatment tool distal end part 13A toward the proximal end side is restricted. Also in the endoscopic treatment tool 1 shown in Fig. 18, the outer surface grooves 32g are formed on the distal end coupling member 32 so that the internal space S is not blocked.

In the endoscopic treatment tool 1 shown in Fig. 18, the protrusion 29 constitutes the first restricting portion that restricts the distal end of the treatment tool distal end part 13A from moving a predetermined distance or more toward the proximal end side with respect to the opening edge 2e in the retracted state.

As described above, at least the following matters are described in the present specification. It should be noted that the constituent elements and the like corresponding to the above-described embodiments are shown in parentheses, but the present invention is not limited thereto.
(1) An endoscopic treatment tool (the endoscopic treatment tool 1) comprising:
   a treatment tool main body (the treatment tool main body 10) of which a distal end part (the treatment tool distal end part 13) is configured to be opened and closed and which extends in a first direction (the axial direction);
   an operation member (the operation member 4) that is provided on a proximal end side of the treatment tool main body and is used for an opening and closing operation of the distal end part; and
   an accommodating member (the accommodating member 2) that has a tubular shape and that accommodates the treatment tool main body inside and has a through-hole (the through-hole 2s) allowing fluid to be supplied inside,
   in which the accommodating member and the treatment tool main body are configured to move relative to each other in the first direction so as to form a first state (the protruding state) in which the treatment tool main body protrudes from a first opening (the distal end opening 2h) provided on a distal end side of the accommodating member or a second state (the retracted state) in which at least a part of a protrusion range of the treatment tool main body in the first state is retracted toward the proximal end side with respect to the first opening.
   According to (1), in the second state, by supplying, for example, a liquid from the through-hole of the accommodating member, the supplied liquid can be discharged from the first opening at the distal end of the accommodating member. Since the liquid can be discharged in a state in which most of the treatment tool main body does not protrude from the first opening of the accommodating member, the liquid can be strongly discharged. In addition, workability of liquid discharge can be improved. In addition, for example, in a case in which the accommodating member itself is configured to move, the above-described effects can be obtained without significantly changing the structure of the existing endoscopic treatment tool, that is, without using a complicated mechanism or the like for adjusting the position of the treatment tool main body.
(2) The endoscopic treatment tool according to (1),
   in which a position of the treatment tool main body with respect to the operation member is fixed,
   the accommodating member is configured to move in the first direction with respect to the operation member.
   According to (2), the effects described in (1) can be obtained without significantly changing the structure of the existing endoscopic treatment tool, that is, without using a mechanism or the like for adjusting the position of the treatment tool main body.
(3) The endoscopic treatment tool according to (2),
   in which the accommodating member is slidably coupled to the operation member.
   According to (3), the accommodating member can be easily held at any position by the frictional force between the accommodating member and the operation member. This allows for various ways of use depending on the treatment.
(4) The endoscopic treatment tool according to (3),
   in which the accommodating member is attachable to and detachable from the operation member.
   According to (4), ways of use depending on the purpose are possible.
(5) The endoscopic treatment tool according to (2), further comprising:
   a coupling member (the coupling member 3) that couples the accommodating member and the operation member to each other and is attachable to and detachable from the operation member,
   in which the accommodating member is slidably coupled to the coupling member.
   According to (5), the accommodating member can be easily held at any position by the frictional force between the accommodating member and the coupling member. This allows for various ways of use depending on the treatment.
(6) The endoscopic treatment tool according to any one of (1) to (5), further comprising:
   a first restricting portion (the restricting member 5, the restricting member 5A, the restricting member 5B, the diameter-reduced portion 2j, the ring member 9, the outer surface recessed portions 2k, the small-diameter member 2n, and the protrusion 29) that restricts a distal end (the distal end 20e) of the treatment tool main body from moving a predetermined distance or more toward the proximal end side with respect to an opening edge (the opening edge 2e) of the first opening.
   According to (6), since the distal end of the treatment tool main body does not move to the proximal end side with respect to the opening edge of the first opening beyond the predetermined distance, the liquid can be discharged from the first opening in a state in which the distance between the first opening of the accommodating member and the distal end of the treatment tool main body is maintained at the optimum position. As a result, the liquid can be discharged with a strong pressure. By ideally setting the predetermined distance to zero, the liquid can be discharged at the strongest pressure.
(7) The endoscopic treatment tool according to (6),
   in which the first restricting portion is provided at the accommodating member.
   According to (7), it is possible to increase the degree of freedom in designing the restricting member.
(8) The endoscopic treatment tool according to (7),
   in which the first restricting portion is provided at a proximal end part (the proximal end part 2a) of the accommodating member.
   According to (8), the restricting member can be easily formed.
(9) The endoscopic treatment tool according to (7),
   in which the first restricting portion is provided at a distal end part (the distal end part 2c) of the accommodating member.
   According to (9), the restriction by the restricting member can be performed with high accuracy.
(10) The endoscopic treatment tool according to any one of (1) to (9), further comprising:
   a sealing member (the sealing member 8) provided between an inner peripheral surface (the inner peripheral surface 2i) of the accommodating member on the proximal end side and an outer peripheral surface (the outer peripheral surface 1 1u) of the treatment tool main body.
   According to (10), it is possible to prevent the liquid supplied to the inside of the accommodating member from leaking to the proximal end side.
(11) The endoscopic treatment tool according to (10),
   in which the sealing member is provided on the proximal end side with respect to the through-hole.
   According to (11), it is possible to prevent the liquid supplied to the inside of the accommodating member from leaking to the proximal end side.
(12) The endoscopic treatment tool according to any one of (1) to (11), further comprising:
   a second restricting portion (the restricting member 5, the restricting member 5A, the restricting member 5B, and the stepped end surface 4f) that restricts a maximum distance (the maximum distance L) between a distal end (the distal end 20e) of the treatment tool main body and an opening edge (the opening edge 2e) of the first opening in the first state to a predetermined value.
   According to (12), since the state in which the distal end of the treatment tool main body protrudes from the accommodating member at an appropriate distance can be maintained, the operation of the distal end of the treatment tool main body can be stably performed.
(13) The endoscopic treatment tool according to any one of (1) to (12),
   in which the operation member includes a main body part (the main body part 4a) and an operation element (the operation element 4b) supported by the main body part so as to be movable in the first direction, and
   the distal end part of the treatment tool main body is configured to be opened and closed in response to movement of the operation element.
   According to (13), advanced treatment is possible.
(14) An endoscopic treatment tool (the endoscopic treatment tool 1) comprising:
   a treatment tool main body (the treatment tool main body 10) that extends in a first direction (the axial direction);
   an operation member (the operation member 4) that is provided on a proximal end side of the treatment tool main body and is used for operating the treatment tool main body; and
   an accommodating member (the accommodating member 2) that has a tubular shape and that accommodates the treatment tool main body inside and has a through-hole (the through-hole 2s) allowing fluid to be supplied inside,
   in which a position of the treatment tool main body with respect to the operation member is fixed,
   the accommodating member is configured to move in the first direction, and a first state (the protruding state) in which the treatment tool main body protrudes from a first opening (the distal end opening 2h) provided on a distal end side of the accommodating member and a second state (the retracted state) in which at least a part of a protrusion range of the treatment tool main body in the first state is retracted toward the proximal end side with respect to the first opening is formed by movement of the accommodating member.
(15) An endoscopic treatment tool (the endoscopic treatment tool 1) comprising:
   a treatment tool main body (the treatment tool main body 10) that extends in a first direction (the axial direction);
   an operation member (the operation member 4) that is provided on a proximal end side of the treatment tool main body and is used for operating the treatment tool main body; and
   an accommodating member (the accommodating member 2) that has a tubular shape and that accommodates the treatment tool main body inside and has a through-hole (the through-hole 2s) allowing fluid to be supplied inside,
   in which the accommodating member and the treatment tool main body are configured to move relative to each other in the first direction so as to form a first state (the protruding state) in which the treatment tool main body protrudes from a first opening (the distal end opening 2h) provided on a distal end side of the accommodating member or a second state (the retracted state) in which at least a part of a protrusion range of the treatment tool main body in the first state is retracted toward the proximal end side with respect to the first opening, and
   the endoscopic treatment tool further comprises a first restricting portion (the restricting member 5, the restricting member 5A, the restricting member 5B, the diameter-reduced portion 2j, the ring member 9, the outer surface recessed portions 2k, the small-diameter member 2n, and the protrusion 29) that restricts a distal end (the distal end 20e) of the treatment tool main body from moving a predetermined distance or more toward the proximal end side with respect to an opening edge (the opening edge 2e) of the first opening.
(16) The endoscopic treatment tool according to (14) or (15),
   in which a distal end part of the treatment tool main body includes a first portion (the trunk portion 13 a) on the proximal end side and a second portion (the leading distal end portion 13b) thicker than the first portion and provided on a distal end side with respect to the first portion.
(17) A water supply member (the accommodating member 2) having a tubular shape into which a treatment tool main body (the treatment tool main body 10) extending in a first direction (the axial direction) is capable of being inserted, the water supply member comprising:
   a through-hole (the through-hole 2s) provided on an outer side surface; and
   a first restricting portion (the restricting member 5, the restricting member 5A, the restricting member 5B, the diameter-reduced portion 2j, the ring member 9, the outer surface recessed portions 2k, the small-diameter member 2n, and the protrusion 29) that restricts a distal end (the distal end 20e) of the inserted treatment tool main body from moving a predetermined distance or more toward a proximal end side with respect to an opening edge (the opening edge 2e) of a first opening (the distal end opening 2h) provided on a distal end side of the water supply member.
(18) The water supply member according to (17), further comprising:
   a sealing member (the sealing member 8) provided between an inner peripheral surface (the inner peripheral surface 2i) on the proximal end side and an outer peripheral surface (the outer peripheral surface 1 1u) of the inserted treatment tool main body.
(19) The water supply member according to (18),
   in which the sealing member is provided on the proximal end side with respect to the through-hole.
(20) The water supply member according to any one of (17) to (19),
   in which the water supply member is slidably coupled to an operation member (the operation member 4) for operating the treatment tool main body.
(21) The water supply member according to any one of (17) to (19), further comprising:
   a coupling member (the coupling member 3) that is attachably and detachably coupled to an operation member (the operation member 4) for operating the treatment tool main body,
   in which the water supply member is slidably coupled to the coupling member.

### Explanation of References

1: endoscopic treatment tool
d1: distance
d2: external dimension
2na, 9a: end surface
2a, 33a: proximal end part
2b: connection protruding portion
2c: distal end part
2d: inner surface stepped portion
2e: opening edge
2g: projecting portion
2h: distal end opening
2i: inner peripheral surface
2j: diameter-reduced portion
2k: outer surface recessed portion
2m: large-diameter member
2n: small-diameter member
4s: internal space
2as: proximal end side space
2s: through-hole
2: accommodating member
3h: opening
3j, 6j coupling recessed portion
3: coupling member
4sa: distal end side end surface
4a, 6b, 6, 7: main body part
4b: operation element
4d: connection terminal
4e: distal end fitting portion
4f: stepped end surface
4k: slide piece portion
4t: main body distal end part
4: operation member
5A, 5B, 5 restricting member
5b: clamping piece
5c: coupling piece
5d: distal end locking piece
5f: flange portion
5t: distal end surface
6a: coupling projecting portion
6bu: outer surface
8A, 8: sealing member
9g: inner surface groove
9: ring member
10: treatment tool main body
11a: cord fixing portion
1 1u: outer peripheral surface
11: flexible cord
13A, 13: treatment tool distal end part
13a: trunk portion
13b: leading distal end portion
20a: sawtooth-shaped uneven portion
20e: distal end
20: forceps piece
21: support shaft
23: link plate portion
24, 26: pivot shaft
25: link rod
27: actuating member
28: attachment member
29: protrusion
30: coil sleeve
31: insulating tube
32a: proximal-end end surface
32g: outer surface groove
32: distal end coupling member
33: operation wire
53: annular plate
61, 62: fixing member
100: liquid supply device
101: supply pipe
200: high-frequency power supply
201, 202: cable
203: facing plate

## Claims

1. An endoscopic treatment tool comprising:
a treatment tool main body of which a distal end part is configured to be opened and closed and which extends in a first direction;
an operation member that is provided on a proximal end side of the treatment tool main body and is used for an opening and closing operation of the distal end part; and
an accommodating member that has a tubular shape and that accommodates the treatment tool main body inside and has a through-hole allowing fluid to be supplied inside,
wherein the accommodating member and the treatment tool main body are configured to move relative to each other in the first direction so as to form a first state in which the treatment tool main body protrudes from a first opening provided on a distal end side of the accommodating member or a second state in which at least a part of a protrusion range of the treatment tool main body in the first state is retracted toward the proximal end side with respect to the first opening.

2. The endoscopic treatment tool according to claim 1,
wherein a position of the treatment tool main body with respect to the operation member is fixed, and
the accommodating member is configured to move in the first direction with respect to the operation member.

3. The endoscopic treatment tool according to claim 2,
wherein the accommodating member is slidably coupled to the operation member.

4. The endoscopic treatment tool according to claim 3,
wherein the accommodating member is attachable to and detachable from the operation member.

5. The endoscopic treatment tool according to claim 2, further comprising:
a coupling member that couples the accommodating member and the operation member to each other and is attachable to and detachable from the operation member,
wherein the accommodating member is slidably coupled to the coupling member.

6. The endoscopic treatment tool according to any one of claims 1 to 5, further comprising:
a first restricting portion that restricts a distal end of the treatment tool main body from moving a predetermined distance or more toward the proximal end side with respect to an opening edge of the first opening.

7. The endoscopic treatment tool according to claim 6,
wherein the first restricting portion is provided at the accommodating member.

8. The endoscopic treatment tool according to claim 7,
wherein the first restricting portion is provided at a proximal end part of the accommodating member.

9. The endoscopic treatment tool according to claim 7,
wherein the first restricting portion is provided at a distal end part of the accommodating member.

10. The endoscopic treatment tool according to any one of claims 1 to 5, further comprising:
a sealing member provided between an inner peripheral surface of the accommodating member on the proximal end side and an outer peripheral surface of the treatment tool main body.

11. The endoscopic treatment tool according to claim 10,
wherein the sealing member is provided on the proximal end side with respect to the through-hole.

12. The endoscopic treatment tool according to any one of claims 1 to 5, further comprising:
a second restricting portion that restricts a maximum distance between a distal end of the treatment tool main body and an opening edge of the first opening in the first state to a predetermined value.

13. The endoscopic treatment tool according to any one of claims 1 to 5,
wherein the operation member includes a main body part and an operation element supported by the main body part so as to be movable in the first direction, and
the distal end part of the treatment tool main body is openable and closable in response to movement of the operation element.

14. An endoscopic treatment tool comprising:
a treatment tool main body that extends in a first direction;
an operation member that is provided on a proximal end side of the treatment tool main body and is used for operating the treatment tool main body; and
an accommodating member that has a tubular shape and that accommodates the treatment tool main body inside and has a through-hole allowing fluid to be supplied inside,
wherein a position of the treatment tool main body with respect to the operation member is fixed,
the accommodating member is configured to move in the first direction, and
a first state in which the treatment tool main body protrudes from a first opening of the accommodating member on a distal end side and a second state in which at least a part of a protrusion range of the treatment tool main body in the first state is retracted toward the proximal end side with respect to the first opening are formed by movement of the accommodating member.

15. An endoscopic treatment tool comprising:
a treatment tool main body that extends in a first direction;
an operation member that is provided on a proximal end side of the treatment tool main body and is used for operating the treatment tool main body; and
an accommodating member that has a tubular shape and that accommodates the treatment tool main body inside and has a through-hole allowing fluid to be supplied inside,
wherein the accommodating member and the treatment tool main body are configured to move relative to each other in the first direction so as to form a first state in which the treatment tool main body protrudes from a first opening provided on a distal end side of the accommodating member or a second state in which at least a part of a protrusion range of the treatment tool main body in the first state is retracted toward the proximal end side with respect to the first opening, and
the endoscopic treatment tool further comprises a first restricting portion that restricts a distal end of the treatment tool main body from moving a predetermined distance or more toward the proximal end side with respect to an opening edge of the first opening.

16. The endoscopic treatment tool according to claim 14 or 15,
wherein a distal end part of the treatment tool main body includes a first portion on the proximal end side and a second portion thicker than the first portion and provided on the distal end side with respect to the first portion.

17. A water supply member having a tubular shape into which a treatment tool main body extending in a first direction is capable of being inserted, the water supply member comprising:
an outer side surface on which a through-hole is provided; and
a first restricting portion that restricts a distal end of the inserted treatment tool main body from moving a predetermined distance or more toward a proximal end side with respect to an opening edge of a first opening provided on a distal end side of the water supply member.

18. The water supply member according to claim 17, further comprising:
a sealing member provided between an inner peripheral surface of the water supply member on the proximal end side and an outer peripheral surface of the inserted treatment tool main body.

19. The water supply member according to claim 18,
wherein the sealing member is provided on the proximal end side with respect to the through-hole.

20. The water supply member according to any one of claims 17 to 19,
wherein the water supply member is slidably coupled to an operation member for operating the treatment tool main body.

21. The water supply member according to any one of claims 17 to 19, further comprising:
a coupling member that is attachably and detachably coupled to an operation member for operating the treatment tool main body,
wherein the water supply member is slidably coupled to the coupling member.
